# EUROPEAN PATENT SPECIFICATION

(11) **EP 1 731 089 B1**
(45) Date of publication and mention of the grant of the patent: **31.12.2008**
(21) Application number: 06252952.4
(22) Date of filing: 07.06.2006
(51) Int. Cl.: A61B 5/00, A61M 16/00

(54) **Multi-part medical system**
Mehrteiliges medizinisches System
Système médical en plusieurs parties

(30) Priority: 08.06.2005 DE 102005026562
(43) Date of publication of application: 13.12.2006
(73) Proprietor: Dräger Medical AG & Co. KG, 23542 Lübeck (DE)
(72) Inventor: Schermeier, Olaf, 23560 Lübeck (DE); Ziermann, Frank, 20146 Hamburg (DE)
(74) Representative: Strauss, Steffen

(56) References cited:
- EP-A- 0 571 225
- WO-A-00/61003
- WO-A-02/02169
- WO-A-93/06776
- WO-A-20/04095379
- DE-A1- 10 021 783
- US-A- 5 910 776
- US-A1- 2002 038 392

## Description

The invention relates to a multi-part medical system with a multi-functional interface.

Medical systems are characterised by increasing complexity and modularity. Before a medical system can be used at all, a plurality of components usually has to be connected. Numerous interfaces usually have to be connected using means of connection, and this can give rise to a high outlay, a high risk of error and possibly problems with availability.

Apart from connections that are required to produce various material flows, increasing importance is being attached to possible ways of creating data connections. During medical treatment, numerous items of information must permanently be kept available. At the same time, an abundance of other data arises during the treatment of patients, and these have to be recorded and/or taken into account in therapeutic decisions. A loss of such data, which can certainly occur under the time pressure that usually prevails, requires increased outlay on data recovery or on further data collection. Numerous data interfaces are known that are connected with separate cables or other means of connection in order to allow medical components to communicate with one another.

Furthermore, it is known to provide external data carriers with data records in order to be able to undertake a speedier adaptation of medical components when the need arises. Thus, for example, it is known to clear or block certain operating or operator modes on medical apparatuses by means of external data carriers (DE 101 16 650 A1). Furthermore, it is known to carry out an adaptation of such apparatuses to the requirements of individual operators by means of external data carriers and the connection thereof to complex, computer-controlled medical equipment (DE 196 25 410 A1).

Finally, it is known to store, on an external data memory, adjustment and parameter sets of medical apparatuses, for example an ECG monitor, that have arisen during a treatment, and to input this memory after replacement of the given apparatus by the interchanged apparatus. The necessity of having to transfer parameter sets and settings manually is thus no longer present (DE 198 09 952 A1).

The common feature with the preceding examples of the prior art is the fact that in each case an external data carrier, which is specially configured for storing and keeping available special data records, has to be connected to a medical apparatus in order to provide the full operational capability. This gives rise on the one hand to separate manoeuvres and requires increased awareness, since such data carriers, usually designed as a chip card, can quickly be lost in working procedures taking place under great pressure of time, which would lead to a considerable hold-up or subsequent expenditure.

The present invention is as claimed in the claims.

The present invention provides a possible way of reducing the risk of incorrect operations or data loss in complex medical systems, without having to incur a greatly increased outlay.

The invention essentially proceeds from two starting points. The first starting point is the fact that numerous multi-part medical systems have connection elements, with the aid of which individual components can be connected together. These connection elements in many cases necessitate precise positioning to permit a connection to be made.

In particular, fluid interfaces often have suitable connection means which are connected together in order to achieve a substance exchange. The requirement for the tightness of such fluid interfaces that usually exists means that they must be positioned very precisely for a correct connection, or they are designed, by means of suitable guide means, in such a way that incorrect positioning is for the most part ruled out.

Another starting point of the invention proceeds from the fact that, in modern medical systems, components are often present that are intended to remain for a lengthy period close to the patient, whereas other components are replaced more often or are connected only for a short time to the patient or to the medical system. If means for data storage are connected to such a component that remains for a lengthy period close to the patient, a loss of this data carrier during the period when the component remains close to the patient is virtually ruled out. Furthermore, if the component that remains close to the patient for a lengthy period is equipped for this purpose with a data carrier which is addressable via an interface mechanically integrated into the already mentioned connection means, it is moreover guaranteed that, in the case of the connected components, the data carrier is on the one hand available and on the other hand the interface for addressing the data carrier is precisely positioned with respect to other components that are connected to the component remaining close to the patient. Particularly precise positioning results when the interface for addressing the data carrier is mechanically integrated into a fluid interface, via which a connection of the component remaining close to the patient with other components of the multi-part medical system takes place.

WO-A-93/06776 discloses a catheter assembly having a catheter with at least one transducer associated therewith for directly measuring physiological parameters of a patients or measuring an amount of a parameter indicative of a physiological condition of the patient and a memory which re sides at a predetermined location on said catheter. The memory contains encoded calibration information for calibrating the transducers and encoded patient specific information which can be accessed by an external processing system to which the catheter assembly is connected for processing. The memory is further designed such that disconnection of the catheter assembly from the external processing system does not cause values stored in the memory to be lost so that the patient specific information need not be reentered into the memory when the catheter assembly is reconnected to the same or another external processing system. By so providing the catheter assembly with memory, information for factory calibration, patient calibration and historical patient data may be stored with the catheter for ease of use. The data in the memory may also be coded to prevent easy replication of the catheter by a competing manufacturer.

WO-A-02/02169 discloses a gas compression and delivery device for treatment of sleep disorders. The device has a motor, at least one impeller, and two air pressure chambers, each receiving air at a different pressure, one pressure applied to a patient during inspiration and one for expiration. A mask having a dual pressure gas delivery hose and a selector for letting in either the high or low pressure gas depending on the breathing cycle of the patient. The gas pressures are adjustable by means of valves on the separate chambers in the device. At least one sensor on the patient sending data to a controller in the device as to the patient's physiological data which is used to determine the patient's breathing treatment needs. The controller may be a microprocessor with memory capability to store patient data for diagnosis and treatment of the patient. Telecommunications by telemetry, or telephony to a remote site allows home use of the device rather than institutional use with healthcare providers on site. A data card may be used to input and or store data in the device. The controller is capable of instructing the device to treat the patient with a number of different protocols and record the patient's physiological data for diagnosis and treatment purposes.

WO-A-2004/095379 discloses a delivery system, comprising dispensing means having an outlet for delivering one or more materials or more articles; dispensing control means for controlling the passage of the material or article through the outlet; first identification means operable for recording, emitting, carrying or associating first identify data to identify the dispensing means, or the material or article carried thereby; and permission control means operable to establish a predetermined condition of the dispensing control means when a corresponding predetermined relationship is established between the first identity data and second identity of an associated entity.

WO-A-00/61003 discloses a multi-part medical system having the features of the preamble of claim 1. The present invention is characterized by the features of the characterizing portion of claim 1. Optional features are recited in the dependent claims.

The invention consists in a multi-part medical system, which includes at least two components connectable via position-determining connection means, whereby at least a first component is intended to remain close to the patient in the connected and in the unconnected state of the system and at least a second component can be removed or replaced by other components in the unconnected state, whereby the first component contains means for data storage, which can be written or read out via an interface which is mechanically integrated into the position-determining connection means. Position-determining connection means is understood to mean means which must occupy a defined position in respect of one another in order to permit the desired connection to be made. The first component is either a respiratory tube system or a mask body.

To advantage, the interface contained according to the invention for addressing the means for data storage may be integrated into a fluid interface. A fluid interface within the meaning of the invention is any connection system which can enter into an interaction via shaped pieces complementary with one another, whereby a sealing effect is achieved in a keyed or friction-locked manner and an exchange of a fluid through the connected interface can take place in the connected state. Typical fluids are respiratory gases or liquids during intensive-therapeutic measures.

The connection means connectable to one another, or more precisely the parts of the fluid interface connectable to one another, are connected at least with sufficient dimensional stability to means for a data transmission in a way which ensures that, in the case of the components of the medical system according to the invention connected to one another, the means of data transmission, which form the interface for addressing the data memory, are at least arranged in such a way that data transmission can take place. This principle of linking means for a data transmission to parts of a fluid interface connectable to one another or other position-determining connection means connectable to one another is understood as integration within the meaning of the invention.

The connection of means for data storage to a component that remains for a lengthy period close to the patient is automatically associated with the fact that no further manoeuvres are required to make the data memory available and the latter can never be forgotten. The effect of integrating the means for data transmission into an interface which in any case has to be connected to complementary parts for the operation of the medical system, furthermore, is that no additional manoeuvres are required for the connection of the means for data storage to a writer or reader unit located in another component of the medical system, and this greatly assists with a procedure under pressure of time.

Components of a medical system according to the invention can be routinely removed from the patient or replaced may, for example, be respiratory apparatuses.

The first components are as a rule designed in such a way that they can be connected without problem via interfaces, in some cases even fluid interfaces, to further components of the medical system.

It is particularly advantageous for the interface according to the invention to be equipped with means for contactless data transmission, which is advantageous especially when dealing with oxygen to avoid any ignition hazard.
It is without doubt advantageous for the means for data storage and/or data transmission to be designed in such a way that they are suitable for the storage and transmission of respiratory parameters, which enables a convenient exchange of equipment, especially when use is being made of varied respiratory apparatuses during the treatment of a patient.

In an advantageous development of a system according to the invention, the means for data storage and/or data transmission are designed in such a way that they are suitable for the storage and transmission of information via the components connected by means of the interface. This is an advantageous variant, for example, in the case of the use of respiratory tube systems which can easily be confused with one another. In this case, the system can automatically recognise the connected type of tube.

In more convenient, multi-part medical systems with an interface according to the invention, the means for data storage and/or data transmission are designed in such a way that they are suitable for the storage and transmission of patient data, therapy data and/or diagnostic data. In this way, the means for data storage can in part assume the function of an electronic patient record and necessary data can automatically be made available to the doctor giving treatment at the time.

It has proved to be particularly advantageous for the means for data transmission and/or data storage to be components of an RFID system.

Alternatively, the means for data transmission and/or data storage can be components of a system which is based on magnetic or optical data storage and/or data transmission. 1-wire technology represents a further advantageous alternative.

In order to prevent unauthorised access to the stored data, it is advantageous for the data to be encoded and only to be made available when read out through a suitable decoding process. For this purpose, it is necessary for means of encoding and decoding the transmitted and/or stored data to be contained.

Furthermore, it is advantageous for means to be contained that make it possible to store manually information which prohibits further use of the component intended to remain with the patient. These include, for example, a manual switch which ensures the transmission and storage of a blocking code when actuation takes place. If this code is read out subsequently, the medical system requests replacement of the blocked components. This can be expedient in the case of unclear infection risks and obvious damage.

Embodiments of the invention will now be described, by way of example only, with reference to the accompanying Figures, of which:
Fig. 1 is a schematic diagram of a multi-part medical system according to the invention in the form of a respiratory system,
Fig. 2 is a schematic block diagram of a multi-part medical system according to the invention,
Fig. 3 is a schematic diagram of a respiratory system according to the invention in the area of the fluid interface,
Fig. 4 is a schematic diagram of the general structure of a multi-part medical system according to the invention.

The respiratory system equipped according to the invention includes a respiratory tube system with a memory element as a component which, within the meaning of the invention, is intended to remain for a lengthy period close to the patient. As components that can be routinely removed from the patient or replaced, respiratory apparatuses are included that have a reader and writer unit, which can communicate with the memory element when the respiratory tube apparatus is connected. This offers numerous advantages over conventional respiratory systems, as explained in the following.

There are a large number of different types of respiratory tube and respiratory tube systems (collectively "respiratory tube means"). Thus, there are single-use and multiple-use tubes, different tube lengths, different diameters, double-tube systems, coaxial tubes, tubes with a semi-permeable membrane for the passage of moisture, tubes heated by electrical heating wires, tubes with temperature sensors and flowmeters.

Many patients are provided with artificial respiration as part of their medical care, whereby different respiratory systems may be used one after the other in the course of the treatment.

Each combination of a specific type of tube with a specific respiratory apparatus requires specific respiratory parameters or excludes other respiratory parameters. Furthermore, respiratory parameters must be selected according to therapeutic aspects. The main parameters are the form of respiration, the oxygen content, the respiratory frequency, where appropriate the stroke volume, the maximum volume, the respiratory pressure and a maximum permissible pressure.

Respiratory systems available at present require that the individual patient respiratory parameters are set manually by the user on each apparatus in order to ensure optimum treatment.

The optimum setting of the parameters depends on a large number of individual patient factors which describe the respiratory requirement. The optimum setting of the respiratory parameters therefore requires a considerable expenditure of time by the staff in attendance.

After the start of the medical care, a patient usually passes through various stations. These may be: rescue vehicle/helicopter, ambulance, induction, OP, emergence, intensive care unit and various in-patient or outpatient forms of transport. If respiration is required for a patient, the parameters have to be reset by the staff for each respiratory system along this chain in the case of conventional systems.

The described expenditure and the risk of errors are reduced considerably by the use of a respiratory system equipped according to the invention. Respiratory parameters can be stored as data records in the memory element, which is integrated into the respiratory tube system. The respiratory tube system remains with the patient when the clinical area or the respiratory apparatus are changed. After another respiratory apparatus has been connected up, these data are available for the newly connected respiratory apparatus, which enables an automatic or semi-automatic setting of the required respiratory parameters. Furthermore, data can be stored concerning forbidden parameters which must not under any circumstances be set when the given type of tube is used, and this reduces considerably the risk of patients receiving incorrect care. In addition to or as an alternative to the respiratory parameters, data concerning treatment that has taken place can be stored in the memory element and subsequently read out for accounting purposes. For example, the performed respiratory minutes can thus be recorded.

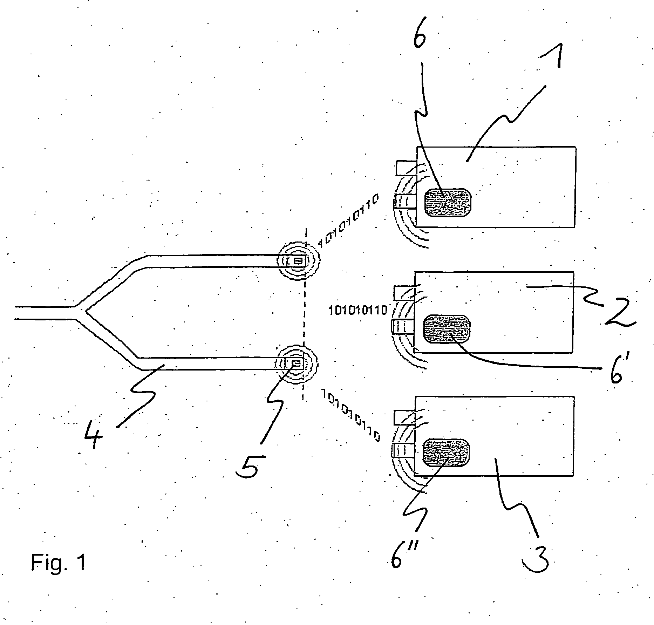

Fig. 1 shows a multi-part medical system according to the invention in the form of a respiratory system. The example of embodiment concerns a system comprising at least two, in the present case three, respiratory apparatuses 1, 2, 3 and at least one respiratory tube system 4, whereby the respiratory apparatuses are capable of storing and reading out in a contactless manner individual patient respiratory parameters on a memory element 5 on respiratory tube system 4 when one of respiratory apparatuses 1, 2, 3 is connected to respiratory tube system 4. The respiratory apparatuses are an emergency respiratory apparatus 1, an intensive-care respiratory apparatus 2 and an anaesthesia respiratory apparatus 3, such as may be used at different times on a patient.

The connection takes place in such a way that respiratory parameters from a respiratory apparatus are stored in each case by a writer and reader unit 6, 6', 6" on memory element 5 of respiratory tube apparatus 4 and these parameters are read out by the other respiratory apparatuses from memory element 5 in respiratory tube system 4 and can thus be set automatically or semi-automatically by the individual respiratory apparatuses. The effect of this is that the respiratory parameters set on the first respiratory apparatus, after re-plugging of the respiratory tube system to another respiratory apparatus, are also set on the latter.

Basic requirements on respiratory tubes are described in EN12342. The standard also defines the mechanical interface to the respiratory system, which is usually designed via a conical male connector to the respiratory system and a female connector to the respiratory tube. The usual standards of 22 mm, 15 mm and 10 mm diameter are available for the connectors. This connector system represents a fluid interface within the meaning of the invention, which in the connected state ensures the precise positioning of the shaped pieces in contact with one another.

Each respiratory apparatus automatically stores all the settings of the respiratory parameters on the memory element in the respiratory tube system. After re-plugging of the tube to another respiratory system, the latter automatically reads out the data last stored on the memory element and sets the latter on the new respiratory system. If necessary, this can take place by retrieval and confirmation on a display screen. If, in turn, any change is made to the settings in the system, this is automatically stored in the memory element and, when the occasion arises, is transferred to another respiratory system. In order not to change the process within the clinical procedure, a passive, cableless memory element is used which can be read out without further work steps.

The advantage of the solution for the user lies in the significant simplification of the clinical processes and thus in a reduction in costs as a result of fewer and shorter work steps.

The expensive manual patient-specific programming of each individual respiratory apparatus is no longer required and is replaced by a brief retrieval. After a change of the clinical area or the respiratory system, the setting of optimum respiratory parameters can be carried out in a matter of a few seconds, something which in conventional systems takes much more time.

Furthermore, optimum treatment of the patient is ensured in all areas, since incorrect operation of the system is largely eliminated. As a result of the further use of optimised respiratory parameters on different apparatuses, it is possible to achieve a stable and lastingly optimised respiratory state.

The communication between the respiratory tube system and the respective respiratory apparatus takes place in the example of embodiment via a contactless data connection.

The memory element takes the form of an RFID chip, a so-called tag, in the tube socket. The former is either glued on or injection moulded. It is arranged geometrically in the tube socket in such a way that it can be read out or written on via an antenna by a writer and reader unit in the respiratory apparatus when the respiratory tube system is connected up to the respiratory apparatus.

RFID performs an inductive process, in which an antenna on a tag is excited at a defined frequency. A small chip on the RFID tag then returns the stored information. There are large number of different RFID standards and RFID tags with varying functionality.

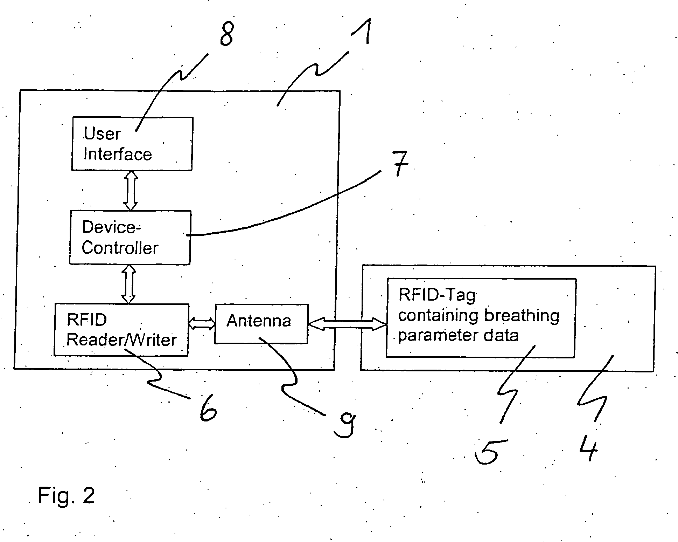

Fig. 2 shows a block diagram of a multi-part medical system according to the invention in the form of a respiratory system. Respiratory apparatus 1 itself contains a control unit 7, which controls all the sequences occurring during the operation of the apparatus. Data required for this can be inputted via an operator unit 8. Respiratory tube system 4 which can be connected to respiratory apparatus 1 has an RFID tag as a memory element 5. A writer and reader unit 6 in respiratory apparatus 1 can communicate with this RFID tag, which takes place via a suitable antenna 9. Writer and reader unit 6 can also relay data read out from the RFID tag to control unit 7. If the RFID tag contains data concerning respiratory parameters, the latter can replace an input via the operator unit. Instead, the read-in respiratory parameters are displayed at operator unit 8 and accepted as a setting by a clearance given by the user.

The RFID tag is already written with various data in the as-delivered state of the respiratory tube system. The latter contain information in the form of an identification number and a manufacturer's code and enable the time of manufacture and other specific data to be read out. Furthermore, respiratory parameters are stored which must not be set with the respiratory tube system. For example, when a respiratory tube system is being used for neonates, it is thus possible to prevent large stroke volumes being set on the respiratory apparatus that would be typical for respiration in the case of adult patients.

If the respiratory apparatus recognises the RFID tag, this means that a tube is connected. Any respiratory parameters already stored on the RFID tag are then regularly compared with the respiratory parameters set in the apparatus software and, when a change has been made by the user, are stored on the RFID tag. Conversely, after the connection of the respiratory tube system to a respiratory apparatus, the stored respiratory parameters are read out from the RFID tag via the writer and reader unit and are used automatically or semi-automatically, after clearance, for the setting of the respiratory mode, which usually takes place through apparatus software.

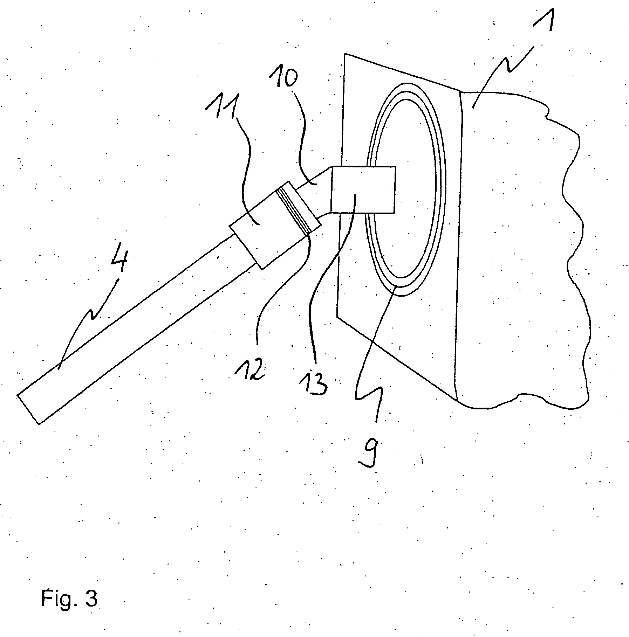

Figure 3 shows a respiratory system according to the invention in the area of the fluid interface. A respiratory gas connection with an angle-adjustable male connector 10 is fitted to a respiratory apparatus 1. The connection of a respiratory tube system 4 is made to this connector 10, whereby a sealing socket 11 as a female connector is connected to male connector 10. An RFID tag, which cannot be seen in the present illustration, is connected to an antenna 12. In the present example, a coil is injection-moulded into socket 11 as antenna 12 of the tag, in such a way that its windings are directed normal to the axis of the tube connection. Antenna 9 of a writer and reader unit on the apparatus side is fitted in this variant at right angles to the axis of the part of respiratory gas connection 13 rigidly connected to respiratory apparatus 1. It thus follows that, with all the positions of the angle-adjustable male connector 10 (except for a connector bent down at right angles), the fields that are formed around antennas 9, 12 exhibit in each case a parallel component with respect to the receiving antenna, which ensures an inductive coupling that is adequate for the performance of the invention.

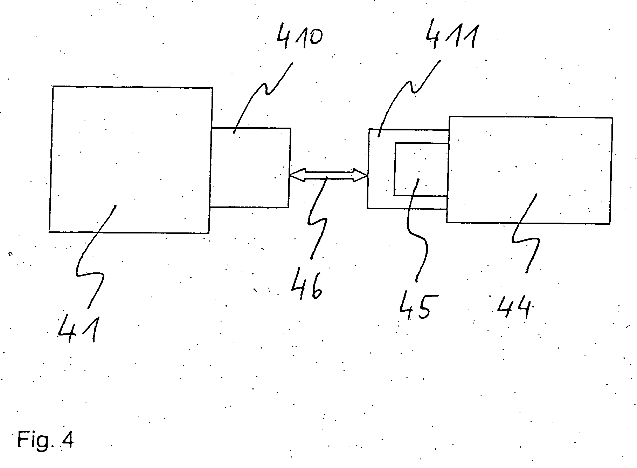

Figure 4 again shows the general structure of a multi-part medical system according to the invention. This is a multi-part medical system which includes at least two components that can be connected via position-determining connection means 410, 411, whereby at least one component 44 is intended to remain close to the patient in the connected and in the unconnected state of the system and at least one component 41 can be removed or replaced by other components in the unconnected state, whereby component 44 intended to remain close to the patient contains means for data storage 45, which can be written and read out via an interface 46 which is integrated mechanically into the position-determining connection means 410, 411.

## Claims

1. A multi-part medical system, which includes at least two components which can be connected via position-determining connection means (410, 411), whereby at least a first component (44) is intended to remain close to the patient in the connected and in the unconnected state of the system and at least second component (41) can be removed or replaced by other components in the unconnected state, the first component (44) containing means for data storage (45) which can be written and read out via an interface (46) which is integrated mechanically into the position-determining connection means (410, 411); the medical system being **characterized in that:**
the first component includes a respiratory tube system (4) or a mask body.

2. The multi-part medical system according to claim 1, in which the connectable components are connectable by position-determining connection means which are a component of a fluid interface.

3. The multi-part medical system according to claim 1 or 2, in which a respiratory apparatus (1) is contained as a component that can be removed from the patient.

4. The multi-part medical system according to any one of claims 1 to 3, in which means for a contactless data transmission are integrated.

5. The multi-part medical system according to any one of claims 1 to 4, in which the means for data storage and/or data transmission are designed in such a way that they are suitable for the storage and transmission of patient data, accounting data, therapy data and/or diagnostic data.

6. The multi-part medical system according to any one of claims 1 to 5, in which the means for data storage and/or data transmission are designed in such a way that they are suitable for the storage and transmission of respiratory parameters.

7. The multi-part medical system according to any one of claims 1 to 5, in which the means for data storage and/or data transmission are designed in such a way that they are suitable for the storage and transmission of information concerning the components connected by means of the interface.

8. The multi-part medical system according to any one of claims 1 to 7, in which the means for data storage and/or data transmission include an RFID system (5, 6, 9, 12).

9. The multi-part medical system according to any one of claims 1 to 7, in which the means for data storage and/or data transmission are a component of a 1-wire system.

10. The multi-part medical system according to any one of claims 1 to 7, in which the means for data storage and/or data transmission are suitable for optical data transmission and/or data storage.

11. The multi-part medical system according to any one of claims 1 to 7, in which the means for data storage and/or data transmission include at least one magnetic storage medium.

12. The multi-part medical system according to any one of claims 1 to 11, in which means for encoding and decoding the transmitted and/or stored data are contained.

13. The multi-part medical system according to any one of claims 1 to 11, in which means are contained which make it possible to store manually information which prohibits further use of the component intended to remain with the patient.

## Patentansprüche

1. Mehrteiliges medizintechnisches System,
welches mindestens zwei über positionsbestimmende Verbindungsmittel (410, 411) verbindbare Komponenten umfasst,
wobei mindestens eine erste Komponente (44) im verbundenen und im nicht verbundenen Zustand des Systems für einen Verbleib in Patientennähe vorgesehen ist und
mindestens eine zweite Komponente (41) im nicht verbundenen Zustand entfernt oder gegen andere Komponenten ausgetauscht werden kann,
wobei die erste Komponente (44) Mittel für eine Datenspeicherung (45) enthält, die über eine Schnittstelle (46) beschrieben und ausgelesen werden können,
wobei die Schnittstelle (46) mechanisch in die positionsbestimmenden Verbindungsmittel (410, 411) integriert ist,
**dadurch gekennzeichnet, dass**
die erste Komponente (44) ein Atemschlauchsystem (4) oder einen Maskenkörper umfasst.

2. Mehrteiliges medizintechnisches System nach Anspruch 1, **dadurch gekennzeichnet, dass** die verbindbaren Komponenten durch positionsbestimmende Verbindungsmittel, die Bestandteil einer fluidischen Schnittstelle sind, verbunden werden können.

3. Mehrteiliges medizintechnisches System nach Anspruch 1 oder 2, **dadurch gekennzeichnet, dass** als eine vom Patienten entfernbare Komponente ein Beatmungsgerät (1) enthalten ist.

4. Mehrteiliges medizintechnisches System nach einem der Ansprüche 1 bis 3, **dadurch gekennzeichnet, dass** Mittel für eine berührungslose Datenübertragung integriert sind.

5. Mehrteiliges medizintechnisches System nach einem der Ansprüche 1 bis 4, **dadurch gekennzeichnet, dass** die Mittel für die Datenspeicherung und / oder Datenübertragung so ausgelegt sind, dass sie für eine Speicherung und Übertragung von Patientendaten, Abrechnungsdaten, Therapiedaten und / oder Diagnostikdaten geeignet sind.

6. Mehrteiliges medizintechnisches System nach einem der Ansprüche 1 bis 5, **dadurch gekennzeichnet, dass** die Mittel für die Datenspeicherung und / oder Datenübertragung so ausgelegt sind, dass sie für eine Speicherung und Übertragung von Beatmungsparametern geeignet sind.

7. Mehrteiliges medizintechnisches System nach einem der Ansprüche 1 bis 5, **dadurch gekennzeichnet, dass** die Mittel für die Datenspeicherung und / oder Datenübertragung so ausgelegt sind, dass sie für eine Speicherung und Übertragung von Informationen über die mittels der Schnittstelle verbundenen Komponenten geeignet sind.

8. Mehrteiliges medizintechnisches System nach einem der Ansprüche 1 bis 7, **dadurch gekennzeichnet, dass** die Mittel für die Datenspeicherung und / oder Datenübertragung ein RFID-System (5, 6, 9, 12) umfassen.

9. Mehrteiliges medizintechnisches System nach einem der Ansprüche 1 bis 7, **dadurch gekennzeichnet, dass** die Mittel für die Datenspeicherung und / oder Datenübertragung Bestandteil eines 1-Wire-Systems sind.

10. Mehrteiliges medizintechnisches System nach einem der Ansprüche 1 bis 7, **dadurch gekennzeichnet, dass** die Mittel für die Datenspeicherung und / oder Datenübertragung für eine optische Datenübertragung und / oder Datenspeicherung geeignet sind.

11. Mehrteiliges medizintechnisches System nach einem der Ansprüche 1 bis 7, **dadurch gekennzeichnet, dass** die Mittel für die Datenspeicherung und / oder Datenübertragung mindestens ein magnetisches Speichermedium umfassen.

12. Mehrteiliges medizintechnisches System nach einem der Ansprüche 1 bis 11, **dadurch gekennzeichnet, dass** Mittel für eine Verschlüsselung und Decodierung der übertragenen und/ oder gespeicherten Daten enthalten sind.

13. Mehrteiliges medizintechnisches System nach einem der Ansprüche 1 bis 11, **dadurch gekennzeichnet, dass** Mittel enthalten sind, die es ermöglichen, manuell eine Information zu speichern, die eine weitere Verwendung der zum Verbleib am Patienten bestimmten Komponente verbietet.

## Revendications

1. Système médical à plusieurs parties, qui comprend au moins deux composants qui peuvent être connectés via des moyens de connexion déterminateurs de position (410, 411), dans lequel au moins un premier composant (44) est destiné à rester près du patient dans l'état connecté et déconnecté du système et au moins un second composant (41) peut être retiré ou remplacé par d'autres composants dans l'état déconnecté, le premier composant (44) contenant des moyens pour le stockage de données (45) qui peuvent être écrites et lues via une interface (46) qui est intégrée mécaniquement dans les moyens de connexion déterminateurs de position (410, 411) ; le système médical étant **caractérisé en ce que :**
le premier composant comprend un système de tube respiratoire (4) ou un corps de masque.

2. Système médical à plusieurs parties selon la revendication 1, dans lequel les composants connectables peuvent être connectés grâce aux moyens de connexion déterminateurs de position qui sont un composant d'une interface fluide.

3. Système médical à plusieurs parties selon la revendication 1 ou 2, dans lequel un appareil respiratoire (1) est contenu sous la forme d'un composant qui peut être retiré du patient.

4. Système médical à plusieurs parties selon l'une quelconque des revendications 1 à 3, dans lequel sont intégrés des moyens destinés à une transmission de données sans contact.

5. Système médical à plusieurs parties selon l'une quelconque des revendications 1 à 4, dans lequel les moyens pour le stockage des données et/ou la transmission des données sont conçus de manière à être adaptés au stockage et à la transmission de données du patient, de données comptables, de données thérapeutiques et/ou de données de diagnostic.

6. Système médical à plusieurs parties selon l'une quelconque des revendications 1 à 5, dans lequel les moyens pour le stockage de données et/ou la transmission de données sont conçus de manière à être adaptés au stockage et à la transmission des paramètres respiratoires.

7. Système médical à plusieurs parties selon l'une quelconque des revendications 1 à 5, dans lequel les moyens pour le stockage de données et/ou la transmission de données sont conçus de manière à être adaptés au stockage et à la transmission d'informations concernant les composants connectés au moyen de l'interface.

8. Système médical à plusieurs parties selon l'une quelconque des revendications 1 à 7, dans lequel les moyens pour le stockage de données et/ou la transmission de données comprennent un système RFID (5, 6, 9, 12).

9. Système médical à plusieurs parties selon l'une quelconque des revendications 1 à 7, dans lequel les moyens pour le stockage de données et/ou la transmission de données sont un composant d'un système à 1 fil.

10. Système médical à plusieurs parties selon l'une quelconque des revendications 1 à 7, dans lequel les moyens pour le stockage de données et/ou la transmission de données sont adaptés pour la transmission de données optiques et/ou le stockage de données.

11. Système médical à plusieurs parties selon l'une quelconque des revendications 1 à 7, dans lequel les moyens pour le stockage de données et/ou la transmission de données comprennent au moins un support de stockage magnétique.

12. Système médical à plusieurs parties selon l'une quelconque des revendications 1 à 11, dans lequel sont contenus des moyens pour coder et décoder les données transmises et/ou stockées.

13. Système médical à plusieurs parties selon l'une quelconque des revendications 1 à 11, dans lequel sont contenus des moyens qui permettent de stocker manuellement des informations qui interdisent en outre l'utilisation du composant destiné à rester avec le patient.
